# EUROPEAN PATENT APPLICATION

(11) **EP 3 415 138 A1**
(43) Date of publication of application: **19.12.2018**
(21) Application number: 17382368.3
(22) Date of filing: 15.06.2017
(51) Int. Cl.: A61K 8/9789, A61K 8/9794, A61K 8/34, A61K 8/60, A61K 8/92, A61K 8/39, A61Q 19/00

(54) **LOTION FOR BALDS**

(71) Applicant: CAROBE'LS COSMETICS, S.L., 24010 San Andrés del Rabanedo (ES)
(72) Inventor: CABERO FERNÁNDEZ, Juan José, 24010 SAN ANDRÉS DEL RABANEDO (ES)
(74) Representative: Manresa Val, Manuel

(57) **Abstract**

It comprises water between 80 and 96% by weight of the total, detergents, between 1.2 and 7% by weight of the total, humectants, between 1 and 6% by weight of the total, castor oil, between 0.1 and 1% by weight of the total, skin conditioning plant extracts, between 0.3 and 3% by weight of the total, toning and refreshing plant extracts, between 0.02 and 2% by weight of the total, preservatives, between 0.2 and 2% by weight of the total, perfume, between 0.1 and 1% by weight of the total, colouring agents, between 0.001 and 0.2% by weight of the total, and pH regulators, between 0.01 and 0.1 % by weight of the total.

## Description

Lotion for balds, comprising water, between 80 and 96% by weight of the total, detergents, between 1.2 and 7% by weight of the total, humectants , between 1 and 6% by weight of the total, castor oil, between 0.1 and 1% by weight of the total, skin conditioning plant extracts, between 0.3 and 3% by weight of the total, toning and refreshing plant extracts, between 0.02 and 2% by weight of the total, preservatives, between 0.2 and 2% by weight of the total, perfume, between 0.1 and 1% by weight of the total, colouring agents, between 0.001 and 0.2% by weight of the total, and pH regulators, between 0.01 and 0.1 % by weight of the total.

### BACKGROUND TO THE INVENTION

Various lotions are known in the state of the art for bald-headed people or for baldness.

European Patent No. 0463937 (ES2062718) "LOTION FOR THE TREATMENT OF ALOPECIA" in the name of JOUVEINAL, S.A., is known in the state of the art, relating to the application of an aqueous lotion of boric acid and salicylic acid and which contains, as a phenolic compound, only one monophenol to prevent hair from falling out or to make it regrow, except for an application as a method for treating the human or animal body or as a diagnosis method applied to the human body.

Also, Spanish Patent ES 2165326 "LOCIÓN CAPILAR PARA LA CURA DE LA ALOPECIA AREATA Y PROCEDIMIENTO DE FABRICACIÓN" (capillary lotion for curing alopecia areata and manufacturing procedure), from the year 2000, in the name of D. Mouhieddine MORAD, is known in the state of the art, which relates to a capillary lotion that cures alopecia areata, and which comprises the following ingredients: common rue (*Ruta graveolens L.*), garlic (*Allium sativum*), garden cress *(Lepidium sativum),* tar, hematites (Fe₂O₃) and phosphorous (P).

### BRIEF DESCRIPTION OF THE INVENTION

This invention is an improvement on the inventions mentioned in the background to the invention.

First of all, this invention differs from the background in that it does not claim that new hair will appear and instead it is aimed at looking after the skin of already bald-headed people.

Even so, regarding the aforementioned documents, the first thereof, European Patent No. 0463937 (ES2062718) comprises boric acid which is highly discussed and controversial compound in the cosmetic sector, and salicylic acid which is a skin exfoliant, the complete opposite of what is intended.

As for Spanish Patent No. ES2165326, among other differences, the use of garlic is strange, since it produces an unpleasant odour in the lotion. It also comprises other ingredients that are forbidden in the cosmetic sector, such as tar, pitch, phosphorous and iron oxides.

This invention has the advantage that it does not use forbidden compositions. Neither does it use compositions which, due to their odour, are not advisable for use.

Also, it is a lotion which, once used, does not need to be rinsed since it is absorbed by the user's own skin, and it has a refreshing and relaxing effect on the skin, it can be applied at any time (i.e., it is not necessary to go into the shower to apply it) and it contains active ingredients that improve blood circulation.

An object of this invention is a lotion for bald-headed people, characterized in that it comprises water, between 80 and 96% by weight of the total, detergents, between 1.2 and 7% by weight of the total, humectants , between 1 and 6% by weight of the total, castor oil, between 0.1 and 1% by weight of the total, skin conditioning plant extracts, between 0.3 and 3% by weight of the total, toning and refreshing plant extracts, between 0.02 and 2% by weight of the total, preservatives, between 0.2 and 2% by weight of the total, perfume, between 0.1 and 1% by weight of the total, colouring agents, between 0.001 and 0.2% by weight of the total, and pH regulators, between 0.01 and 0.1 % by weight of the total.

### SPECIFIC EMBODIMENT OF THIS INVENTION

So, in a specific embodiment of this invention, basically the invention comprises the following formulation:
Water, between 80 and 96% by weight of the total, which is the basic element of the composition.

Some detergents, in a proportion of between 1.2 to 7% by weight of the total. Optionally these detergents may comprise surfactants that are known to be soft, which are the ones that prevent damage to proteins and lipids. Also, they do not dry out the skin. Optionally, the surfactants known to be soft comprise at least one of betaines, amphodiacetanes, glycosides, polyene glycol derivatives, sarcosinates or isethionates.

A humectants, in a proportion of between 1 to 6% by weight of the total, such as for example glycerine, which helps to maintain the skin's humidity, by keeping it duly hydrated.

Castor oil, in a proportion of between 0.1 and 1% by weight of the total, which acts as a moisturising and humectants and it also regenerates the hair in those cases where alopecia is not definitive.

Some skin conditioning plant extracts, in a proportion of between 0.3 and 3% by weight of the total, which condition the skin so that it retains the water better and therefore they keep the skin hydrated. They can be thyme, aloe vera or hamamelis (witch hazel), etc.

Some toning and refreshing plant extracts, in a proportion of between 0.02 and 2% by weight of the total, which tone and refresh the skin as the shampoo is applied. For example, they can be menthol or eucalyptus.

Some preservatives, in a proportion of between 0.2 and 2% by weight of the total, so that the shampoo does not deteriorate. So, for example, a combination of caprylyl glycol with potassium sorbate and a chelating agent or phenoxyethyl alcohol will be used.

A perfume, in a proportion of between 0.1 and 1% by weight of the total.

Some colouring agents, in a proportion of between 0.001 and 0.2% by weight of the total, and

Some pH regulators, in a proportion of between 0.01 and 0.1% by weight of the total. The shampoo must have a pH between 5.5 and 6.5. To this end, for example, it is employed lactic acid, citric acid or phosphoric acid.

A composition example would be as follows:

| **INGREDIENT** | **%** |
|---|---|
| WATER | q.s. 100 |
| GLYCERINE | 5.0 |
| DECYL GLUCOSIDE | 2.0 |
| PHENOXYETHANOL | 0.5 |
| CAPRYLYL GLYCOL | 0.3 |
| PEG-40 HYDROGENATED CASTOR OIL | 0.3 |
| PEG-7 GLYCERYL COCOATE | 0.3 |
| ALOE BARBADENSIS EXTRACT | 0.3 |
| HAMAMELIS VIRGINIANA (WITCH HAZEL) EXTRACT | 0.2 |
| THYMUS VULGARIS (THYME) EXTRACT | 0.2 |
| MENTHOL | 0.06 |
| EUCALYPTOL | 0.02 |
| PERFUME/FRAGRANCE | 0.3 |
| POTASSIUM SORBATE | 0.1 |
| TETRASODIUM EDTA | 0.05 |
| CITRIC ACID | 0.05 |
| C.I. 14720 | 0.001 |
| C.I. 15985 | 0.001 |

The known abbreviation "q.s." which signifies sufficient amount ("*quantum sufficit")* means that it is filled with water until the total is reached, i.e. 100%. In this case, it would be 90.318% water, but to avoid giving so many decimal, in the cosmetic sector, the abbreviation "q.s." is used.

The present invention discloses a new lotion for balds. The examples mentioned herein are non-limiting of this invention, therefore it can have different applications and/or adaptations, all within the scope of the following claims.

## Claims

1. Lotion for balds, **characterized in that** it comprises:
- Water, between 80 and 96% by weight of the total,
- Detergents, between 1.2 and 7% by weight of the total,
- Humectants, between 1 and 6% by weight of the total,
- Castor oil, between 0.1 and 1% by weight of the total,
- Skin conditioning plant extracts, between 0.3 and 3% by weight of the total,
- Toning and refreshing plant extracts, between 0.02 and 2% by weight of the total,
- Preservatives, between 0.2 and 2% by weight of the total,
- Perfume, between 0.1 and 1 % by weight of the total,
- Colouring agents, between 0.001 and 0.2% by weight of the total,
- pH regulators, between 0.01 and 0.1 % by weight of the total.

2. Lotion, according to claim 1, **characterized in that** the detergents comprise surfactants known to be soft.

3. Lotion according to claim 2, **characterized in that** among the surfactants known to be soft at least one of the anionic surfactants, amphoteric surfactants, glycosides, polyene glycol derivatives, sarcosinates or isethionates are comprised.

4. Lotion, according to claim 1, **characterized in that** among the skin conditioning plant extracts at least one of thyme, aloe vera and hamamelis are comprised.

5. Lotion, according to claim 1, **characterized in that** among the toning and refreshing plant extracts at least one of menthol and eucalyptus are comprised.

6. Lotion, according to claim 1, **characterized in that** among the preservatives at least a combination of caprylyl glycol with potassium sorbate and a chelating agent or phenoxyethyl alcohol are comprised.

7. Lotion, according to claim 1, **characterized in that** among the pH regulators at least one of lactic acid, citric acid or phosphoric acid are comprised.
